(19) Europäisches Patentamt
European Patent Office
Office européen des brevets

(11) EP 2 746 388 A2

(12) **EUROPEAN PATENT APPLICATION**
published in accordance with Art. 153(4) EPC

(43) Date of publication:
**25.06.2014 Bulletin 2014/26**

(21) Application number: **12826123.7**

(22) Date of filing: **20.08.2012**

(51) Int Cl.:
***C12N 7/01*** (2006.01) ***A61K 35/76*** (2006.01)
***A61P 35/00*** (2006.01)

(86) International application number:
**PCT/KR2012/006610**

(87) International publication number:
**WO 2013/027988 (28.02.2013 Gazette 2013/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.08.2011 KR 20110083150**

(71) Applicant: **National Cancer Center**
**Goyang-si, Gyeonggi-do 410-769 (KR)**

(72) Inventor: **LEE, Sang Jin**
**Goyang-si**
**Gyeonggi-do 411-310 (KR)**

(74) Representative: **Bockhorni & Kollegen**
**Elsenheimerstraße 49**
**80687 München (DE)**

# (54) RECOMBINANT ADENOVIRUS COMPRISING TRANS-SPLICING RIBOZYME AND CANCER-TREATING GENE, AND USE THEREOF

(57) The present invention relates to: a recombinant adenovirus comprising a polynucleotide, which encodes a trans-splicing ribozyme-HSVtk composite relating to a cancer-specific gene, and a cancer-treating gene; a pharmaceutical composition for preventing or treating cancer, containing the recombinant adenovirus as an active ingredient; and a method for treating cancer, comprising the step of administering the recombinant adenovirus or the pharmaceutical composition to an individual requiring treatment. The recombinant adenovirus of the present invention shows selectivity for a cancer cell by a trans-splicing ribozyme relating to a cancer-specific gene and an increased anticancer activity by a cancer-treating gene, and thus can be widely used for the effective prevention and treatment of cancer.

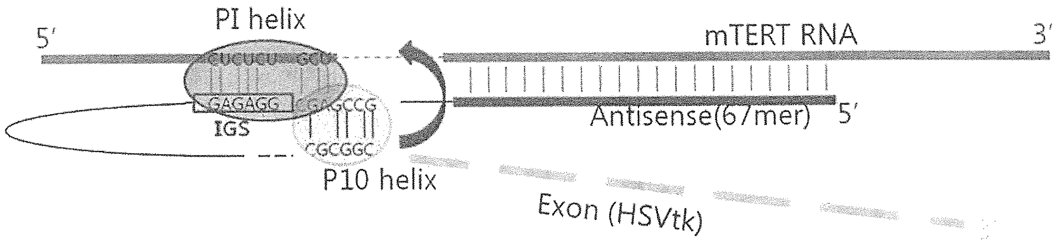


Fig. 1



Printed by Jouve, 75001 PARIS (FR)

<110> NATIONAL CANCER CENTER

<120> Recombinant adenovirus comprising trans-splicing ribozyme and therapeutic genes having anti-cancer activity and the Use thereof

<130> OPA12106/PCT

<150> 10-2011-0083150
<151> 2011-08-19

<160> 6

<170> KopatentIn 2.0

<210> 1
<211> 3210
<212> DNA
<213> Artificial Sequence

<220>
<223> TERT

<400> 1
atgccgcgcg ctccccgctg ccgagccgtg cgctccctgc tgcgcagcca ctaccgcgag 60

gtgctgccgc tggccacgtt cgtgcggcgc ctggggcccc agggctggcg gctggtgcag 120

cgcggggacc cggcggcttt ccgcgcgctg gtggcccagt gcctggtgtg cgtgccctgg 180

gacgcacggc cgccccccgc cgccccctcc ttccgccagg tgtcctgcct gaaggagctg 240

gtggcccgag tgctgcagag gctgtgcgag cgcggcgcga agaacgtgct ggccttcggc 300

ttcgcgctgc tggacggggc ccgcgggggc ccccccgagg ccttcaccac cagcgtgcgc 360

agctacctgc ccaacacggt gaccgacgca ctgcggggga gcggggcgtg ggggctgctg 420

ctgcgccgcg tgggcgacga cgtgctggtt cacctgctgg cacgctgcgc gctctttgtg 480

ctggtggctc ccagctgcgc ctaccaggtg tgcgggccgc cgctgtacca gctcggcgct 540

gccactcagg cccggccccc gccacacgct agtggacccc gaaggcgtct gggatgcgaa 600

cgggcctgga accatagcgt cagggaggcc ggggtccccc tgggcctgcc agccccgggt 660

gcgaggaggc gcgggggcag tgccagccga agtctgccgt tgcccaagag gcccaggcgt 720

ggcgctgccc ctgagccgga gcggacgccc gttgggcagg ggtcctgggc ccacccgggc 780

aggacgcgtg gaccgagtga ccgtggtttc tgtgtggtgt cacctgccag acccgccgaa 840

gaagccacct ctttggaggg tgcgctctct ggcacgcgcc actcccaccc atccgtgggc 900

cgccagcacc acgcgggccc cccatccaca tcgcggccac cacgtccctg ggacacgcct 960

tgtcccccgg tgtacgccga gaccaagcac ttcctctact cctcaggcga caaggagcag 1020

ctgcggccct ccttcctact cagctctctg aggcccagcc tgactggcgc tcggaggctc 1080

gtggagacca tctttctggg ttccaggccc tggatgccag ggactccccg caggttgccc 1140

cgcctgcccc agcgctactg gcaaatgcgg cccctgtttc tggagctgct tgggaaccac 1200

gcgcagtgcc cctacggggt gctcctcaag acgcactgcc cgctgcgagc tgcggtcacc 1260

ccagcagccg gtgtctgtgc ccgggagaag ccccagggct ctgtggcggc ccccgaggag 1320

gaggacacag acccccgtcg cctggtgcag ctgctccgcc agcacagcag cccctggcag 1380

gtgtacggct tcgtgcgggc ctgcctgcgc cggctggtgc ccccaggcct ctggggctcc 1440

aggcacaacg aacgccgctt cctcaggaac accaagaagt tcatctccct ggggaagcat 1500

gccaagctct cgctgcagga gctgacgtgg aagatgagcg tgcgggactg cgcttggctg 1560

cgcaggagcc caggggttgg ctgtgttccg gccgcagagc accgtctgcg tgaggagatc 1620

ctggccaagt tcctgcactg gctgatgagt gtgtacgtcg tcgagctgct caggtctttc 1680

ttttatgtca cggagaccac gtttcaaaag aacaggctct ttttctaccg gaagagtgtc 1740

tggagcaagt tgcaaagcat tggaatcaga cagcacttga agagggtgca gctgcgggag 1800

ctgtcggaag cagaggtcag gcagcatcgg gaagccaggc ccgccctgct gacgtccaga 1860

ctccgcttca tccccaagcc tgacgggctg cggccgattg tgaacatgga ctacgtcgtg 1920

ggagccagaa cgttccgcag agaaaagagg gccgagcgtc tcacctcgag ggtgaaggca 1980

ctgttcagcg tgctcaacta cgagcgggcg cggcgccccg gcctcctggg cgcctctgtg 2040

ctgggcctgg acgatatcca cagggcctgg cgcaccttcg tgctgcgtgt gcgggcccag 2100

gacccgccgc ctgagctgta ctttgtcaag gtggatgtga cgggcgcgta cgacaccatc 2160

ccccaggaca ggctcacgga ggtcatcgcc agcatcatca aaccccagaa cacgtactgc 2220

gtgcgtcggt atgccgtggt ccagaaggcc gcccatgggc acgtccgcaa ggccttcaag 2280

agccacgtct ctaccttgac agacctccag ccgtacatgc gacagttcgt ggctcacctg 2340

caggagacca gcccgctgag ggatgccgtc gtcatcgagc agagctcctc cctgaatgag 2400

gccagcagtg gcctcttcga cgtcttccta cgcttcatgt gccaccacgc cgtgcgcatc 2460

aggggcaagt cctacgtcca gtgccagggg atcccgcagg gctccatcct ctccacgctg 2520

ctctgcagcc tgtgctacgg cgacatggag aacaagctgt ttgcggggat tcggcgggac 2580

gggctgctcc tgcgtttggt ggatgatttc ttgttggtga cacctcacct cacccacgcg 2640

aaaaccttcc tcagctatgc ccggacctcc atcagagcca gtctcacctt caaccgcggc 2700

ttcaaggctg ggaggaacat gcgtcgcaaa ctctttgggg tcttgcggct gaagtgtcac 2760

agcctgtttc tggatttgca ggtgaacagc ctccagacgg tgtgcaccaa catctacaag 2820

atcctcctgc tgcaggcgta caggtttcac gcatgtgtgc tgcagctccc atttcatcag 2880

caagtttgga agaaccccac atttttcctg cgcgtcatct ctgacacggc ctccctctgc 2940

tactccatcc tgaaagccaa gaacgcaggg atgtcgctgg gggccaaggg cgccgccggc 3000

cctctgccct ccgaggccgt gcagtggctg tgccaccaag cattcctgct caagctgact 3060

cgacaccgtg tcacctacgt gccactcctg ggtcactca ggacagccca gacgcagctg 3120

agtcggaagc tcccggggac gacgctgact gccctggagg ccgcagccaa cccggcactg 3180

ccctcagact tcaagaccat cctggactga 3210

<210> 2
<211> 397
<212> DNA
<213> Artificial Sequence

<220>
<223> Rib67

<400> 2
gaaaagttat caggcatgca cctggtagct agtctttaaa ccaatagatt gcatcggttt 60

aaaaggcaag accgtcaaat tgcgggaaag gggtcaacag ccgttcagta ccaagtctca 120

ggggaaactt tgagatggcc ttgcaaaggg tatggtaata agctgacgga catggtccta 180

accacgcagc caagtcctaa gtcaacagat cttctgttga tatggatgca gttcacagac 240

taaatgtcgg tcggggaaga tgtattcttc tcataagata tagtcggacc tctccttaat 300

gggagctagc ggatgaagtg atgcaacact ggagccgctg ggaactaatt tgtatgcgaa 360

agtatattga ttagttttgg agtactcgcg cggccgc 397

<210> 3
<211> 1127
<212> DNA
<213> Artificial Sequence

<220>
<223> HSVtk

<400> 3
atggcttcgt accccctgcca tcaacacgcg tctgcgttcg accaggctgc gcgttctcgc 60

ggccatagca accgacgtac ggcgttgcgc cctcgccggc agcaagaagc cacggaagtc 120

cgcctggagc agaaaatgcc cacgctactg cgggtttata tagacggtcc tcacgggatg 180

gggaaaacca ccaccacgca actgctggtg gccctgggtt cgcgcgacga tatcgtctac 240

gtacccgagc cgatgactta ctggcaggtg ctgggggctt ccgagacaat cgcgaacatc 300

tacaccacac aacaccgcct cgaccagggt gagatatcgg ccggggacgc ggcggtggta 360

atgacaagcg cccagataac aatgggcatg ccttatgccg tgaccgacgc cgttctggct 420

cctcatatcg gggggggaggc tgggagctca catgccccgc ccccggccct caccctcatc 480

ttcgaccgcc atcccatcgc cgccctcctg tgctacccgg ccgcgcgata ccttatgggc 540

agcatgaccc cccaggccgt gctggcgttc gtggccctca tcccgccgac cttgtccggc 600

acaaacatcg tgttgggggc ccttccggag gacagacaca tcgaccgcct ggccaaacgc 660

cagcgccccg gcgagcggct tgacctggct atgctggccg cgattcgccg cgtttacggg 720

ctgcttgcca atacggtgcg gtatctgcag ggcggcgggt cgtggcggga ggattgggga 780

cagctttcgg ggacggccgt gccgccccag ggtgccgagc cccagagcaa cgcgggccca 840

cgaccccata tcggggacac gttatttacc ctgtttcggg cccccgagtt gctggccccc 900

aacggcgacc tgtacaacgt gtttgcctgg gccttggacg tcttggccaa acgcctccgt 960

cccatgcacg tctttatcct ggattacgac caatcgcccg ccggctgccg ggacgccctg 1020

ctgcaactta cctccgggat ggtccagacc cacgtcacca cccccggctc cataccgacg 1080

atctgcgacc tggcgcgcac gtttgcccgg gagatggggg aggctaa 1127

<210> 4
<211> 500
<212> DNA
<213> Artificial Sequence

<220>
<223> sPD-1

<400> 4
atgcagatcc cacaggcgcc ctggccagtc gtctgggcgg tgctacaact gggctggcgg 60

ccaggatggt tcttagactc cccagacagg ccctggaacc cccccacctt ctccccagcc 120

ctgctcgtgg tgaccgaagg ggacaacgcc accttcacct gcagcttctc caacacatcg 180

gagagcttcg tgctaaactg gtaccgcatg agccccagca accagacgga caagctggcc 240

gccttccccg aggaccgcag ccagcccggc caggactgcc gcttccgtgt cacacaactg 300

cccaacgggc gtgacttcca catgagcgtg gtcagggccc ggcgcaatga cagcggcacc 360

tacctctgtg gggccatctc cctggccccc aaggcgcaga tcaaagagag cctgcgggca 420

gagctcaggg tgacagagag aagggcagaa gtgcccacag cccaccccag cccctcaccc 480

aggccagccg gccagttcca 500

<210> 5
<211> 39
<212> DNA
<213> Artificial Sequence

<220>
<223> primer

<400> 5
ccgctcgagc tcaccatgtg ggtccggcag gtaccctgg 39

<210> 6
<211> 42
<212> DNA

<213> Artificial Sequence

<220>
<223> primer

<400> 6
agatcttcct cctcctcctt gaaaccggcc ttctggtttg gg 42

**Description**

**Technical Field**

The present invention relates to a recombinant adenovirus, which comprises a trans-splicing ribozyme and a cancer therapeutic gene, and the use thereof, and more particularly to a recombinant adenovirus comprising a polynucleotide encoding a trans-splicing ribozyme-HSVtk complex, which acts against a cancer-specific gene, and a cancer therapeutic gene, a pharmaceutical composition for preventing or treating cancer, which comprises the recombinant adenovirus as an active ingredient, and a method for treating cancer, which comprises administering the recombinant adenovirus or the pharmaceutical composition to a subject in need of treatment.

**Background Art**

Cancer is the first leading cause of death in Korea and is an incurable disease that has not yet been conquered, even though there have been many studies on the conquest of cancer. Conventional therapeutic methods against cancer include surgery, chemotherapy and radiotherapy. However, each of these methods has many limitations, and for this reason, in recent years, other therapeutic methods based on a concept different from that of such therapeutic methods have been studied. Among them, gene therapy has been actively studied.

As used herein, the term "gene therapy" refers to a method of treating inherited or acquired genetic abnormalities, which are difficult to treat by conventional methods, using genetic engineering methods. Specifically, gene therapy comprises administering genetic materials such as DNA and RNA into the human body to express therapeutic proteins or inhibit the expression of specific proteins in order to treat and prevent inherited or acquired genetic defects, viral diseases, or chronic diseases such as cancer or cardiovascular diseases. Gene therapy can fundamentally treat diseases by analyzing the causes of diseases on a genetic basis, and thus is expected to be used to treat incurable diseases and as an alternative to conventional therapeutic methods.

Gene therapies against cancer can be classified into an immunological gene therapy method that induces immune responses in the human body, and a gene therapy method that causes genes to directly disrupt or kill cancer cells. In the latter case, the role of vectors that delivery and express genes in cells is very important. An adenovirus vector has been recognized as one of the most promising vectors for gene therapy, due to its high efficiency of gene delivery, its ability to deliver a gene into undifferentiated cells, and easy production of high-titer virus stocks.

Adenovirus vectors for gene therapy that are generally used are constructed by deleting a series of genes essential for replication and introducing a cytomegalovirus (CMV) or Rous sarcoma virus promoter (RSV) having high promoter activity in order to express a therapeutic protein with high efficiency in *vivo.*

In recent years, cancer tissue-targeted therapy has been attempted in an effort to reduce side effects that occur because a number of target genes that can be used in gene therapy are also expressed in normal cells that undergo significant cell division (Fukuzawa et al., Cancer Res 64: 363-369, 2004). For this therapy, a method of using a tissue-specific promoter instead of CMV or RSV has been proposed, but was not put to practical use because of its low therapeutic efficacy, even though the specificity increases.

To overcome the above-described disadvantage, studies have recently been conduct to develop a tissue-specific adenovirus for cancer therapy using factors other than a tissue-specific promoter. As a typical example, methods of using trans-splicing ribozyme or the like have been developed.

Studies on the development of tissue-specific adenovirus for cancer therapy using trans-splicing ribozyme revealed that a group I intron ribozyme from *Tetrahymena thermophila* can link two separate transcripts to each other by a trans-splicing reaction not only *in vitro,* but also in bacterial cells and human cells.

Specifically, trans-splicing ribozyme based on this group I intron can target a disease-related gene transcript or a specific RNA that is specifically expressed only in diseased cells, thereby reprogramming the RNA to restore to normal RNA or the gene transcript to be replaced with a new therapeutic gene transcript. Thus, it is expected that the trans-splicing ribozyme can be a disease-specific and safe gene therapy technology. In addition, the trans-splicing ribozyme can significantly increase therapeutic effects, because it can remove disease-specific RNA and induce the expression of a desired therapeutic gene product.

In recent studies, since a trans-splicing ribozyme that targets hTERT (human telomerase reverse transcriptase) capable of acting specifically on cancer tissue was known, attempts to develop cancer therapeutic agents using this trans-splicing ribozyme have been actively made, but positive results have not yet been reported.

Known therapeutic genes that are used in gene therapy include herpes simplex thymidine kinase (hereinafter abbreviated as HSVtk), *E. coli* cytosine deaminase (CD), and E. *coli* purine nucleoside phosphorylase (hereinafter abbreviated as PNP) genes. Gene therapies that use these genes are called "gene-directed enzyme prodrug therapies" (GDEPTs) and commonly use prodrugs. Prodrugs that are used in GDEPTs include ganciclovir (GCV), 5-fluorouracil (5-FU) and 6-methylpurine-2-deoxyriboside (6-MeP-dR), which are used together with HSV-TK, CD and PNP, respectively. Non-cytotoxic prodrugs are converted to cytotoxic drugs by introduced genes and are activated mainly by phosphorylation. The advantage of gene therapy that uses suicide genes is that the so-called bystander effect of killing adjacent cells by drugs activated in cells introduced with these genes is significant. This gene therapy seems to be the best strategy that can be applied in a state in which the efficiency of introduction of genes is low.

It is well known to use HSVtk in this gene therapy strategy. Specifically, a HSVtk/GCV system is one of the most widely used methods among suicide gene therapies and is disclosed in WO 90/07936, US 5,837,510, US 5,861,290, WO 98/04290, WO 97/37542 and US 5,631,236. Cells that express HSVtk gene can phosphorylate ganciclovir (GCV), and as a result, cell death can be induced by interference with DNA replication. Currently, this method is being used in 30 kinds or more of clinical trials for gene therapy of various human cancers. However, this method also has disadvantages in that only cells that are proliferating can be killed, the bystander effect is not sufficient, and a cytotoxicity problem can be caused when the prodrug is used in large amounts. For this reason, studies focused on treating cancer using two or more prodrugs in order to improve the cell death effect and the bystander effect have been actively conducted.

Meanwhile, PD-1 (programmed death-1) is a 55 kDa type I transmembrane protein of the Ig gene superfamily and is known as a co-inhibitory molecule on T cells. In other words, PD-1 is a member of the co-inhibitory molecules of the CD28 family of receptors (e.g., CD28, CTLA-4, ICOS and BTLA) that are expressed on activated B cells, T cells and bone marrow cells. Ligands for PD-1 include PD-L1 and PD-L2, which are known to down-regulate the activation of T cells when binding to PD-1. In conventional T cells, PD-1 is not expressed on naive T cells, but is inducibly expressed after T cell activation. In addition, PD-L1 is found at high levels in various human cancers, and the interaction between PD-1 and PD-L1 transduces stimulatory or inhibitory signals into T cells. In other words, the interaction between PD-1 and PD-L1 induces a decrease in the level of tumor invasive lymphocytes and in the T cell receptor-mediated proliferation and causes the immune evasion of tumor cells. Thus, in recent years, studies have been conducted to treat cancer by blocking the signaling of PD-1 or PD-L1 to effectively induce anticancer immune responses.

## Disclosure

### Technical Problem

The present inventors have made extensive efforts to develop a gene therapy method for cancer treatment, which has improved tissue specificity and improved therapeutic efficacy. As a result, the present inventors have found that a recombinant adenovirus designed to include a polynucleotide, which encodes a cancer tissue-specific trans-splicing ribozyme-HSVtk (Human Simplex Virus thymidine kinase) complex, and the cancer therapeutic gene sPD-1, can exhibit an excellent therapeutic effect specific for cancer tissue, and it can also significantly reduce side effects caused by gene therapy, thereby completing the present invention.

### Technical Solution

It is an object of the present invention to provide a recombinant adenovirus including: a polynucleotide encoding a trans-splicing ribozyme-HSVtk complex, which acts against a cancer-specific gene; and a cancer therapeutic gene.

Another object of the present invention is to provide a pharmaceutical composition for preventing or treating cancer, which comprises the recombinant adenovirus as an active ingredient.

Still another object of the present invention is to provide a method for treating cancer in animals excluding humans, the method comprising administering the recombinant adenovirus or the pharmaceutical composition to a subject in need of treatment.

### Advantageous Effects

The recombinant adenovirus of the present invention shows selectivity for cancer cells by the trans-splicing ribozyme, which acts against a cancer-specific gene, and exhibits increased anticancer activity by the cancer therapeutic gene. Thus, it can be effectively used for the prevention and treatment of cancer.

### Description of Drawings

FIG. 1 is a schematic view showing a trans-splicing ribozyme (mTERT-TR)-HSVtk that targets mouse TERT. The region that targets the mouse TERT (mTERT) transcript is indicated by a sequence near the splicing region. The trans-splicing ribozyme specifically recognizes mTERT RNA using the antisense mTERT RNA sequence and cleaves mTERT RNA at the 3' end of IGS (internal-guided-sequence). The 5' end of the HSVtk-encoding RNA of the trans-splicing ribozyme is linked to the cleaved end of mTERT RNA. The possible nucleotide linage between mTERT-targeting mRNA and ribozyme is indicated by vertical lines.

FIG. 2 shows the effect of mouse TERT-TR on the regulation of expression of adenovirus HSVtk gene. FIG. 2(a) shows the results of expressing HSVtk by the E1/E3-deleted adenovirus Ad5mTR under the control of mouse TERT-TR. Ad5MOCK is a control of the E1/E3-deleted adenovirus. FIG. 2(b) shows the results obtained by inoculating CT26 cells ($3 \times 10^3$) into a 96-well plate, and then exposing the cells to various MOIs of Ad5MOCK or Ad5mTR in the presence of 100 $\mu$M GCV. It shows the results of measuring cytotoxicity using a cell proliferation assay kit at 3 days after infection. The data are expressed as mean $\pm$ standard deviation for three independent experiments. FIG. 2(c) shows the results obtained by injecting $5 \times 10^8$ PFU of Ad5MOCK or Ad5mTR into BALB/c mice, injecting GCV (75 mg/kg) intraperitoneally into the mice twice a day, and inoculating CT26 cells ($1 \times 10^6$) subcutaneously into both flanks of the mice. The data are expressed as mean $\pm$ SEM (n=5).

FIG. 3 shows an improved DC-mediated antigen presenting effect induced by HSVtk. In FIG. 3(a), Ad5MOCK or Ad5mTR was injected into an E.G7 tumor in C57/BL6 mice. After 4 days, the tumor was collected and histologically analyzed by H&E staining. In FIG. 3(b), at 2 days after injection with the adenovirus, DCs ($5 \times 10^4$) were collected from the draining lymph nodes around the E.G7 tumor by MACS chromatography using anti-CD11c microbeads, and then co-cultured with OVA-specific CD8 T cells from OT-1 transgenic mice. After 72 hours, the culture supernatant was collected and the amount of IFN-$\gamma$ therein was measured. DC-Ad5MOCK indicates DCs collected from mice injected with Ad5MOCK, and CD-Ad5mTR indicates DCs from mice injected with Ad5mTR. The data are expressed as mean $\pm$ standard deviation values.

FIG. 4 shows a method for producing a dual-module adenovirus that expresses mTERT-TR-controlled HSVtk and sPD1-Ig. In FIG. 4(a), Ad5mTR.sPD1 encodes mTERT-TR-HSVtk under the control of CMV promoter in the E1 region and encodes sPD1-Ig under the control of EF1$\alpha$ in the E3 region. In FIG. 4(b), HEK293 cells ($4 \times 10^5$) were infected with 2 MOI of adenovirus for 48 hours, disrupted with RIPA buffer, and then analyzed by immunoblotting. The expression of sPD1-Ig by Ad5mTR.sPD1 (80 $\mu$g protein per lane) was analyzed using anti-PD1 antibody. In FIG. 4(c), the enzymatic activity of HSVtk in phosphorylated GCV was evaluated in CT26 cells by measuring the accumulation of radioisotope-labeled PCV (1 $\mu$Ci/ml). The data are expressed as mean $\pm$ standard deviation values for three independent experiments. In FIG. 4(d), the culture supernatant collected from the cells infected with 10 MOI of adenovirus was mixed with a co-culture of MC38/OVA cells ($1 \times 10^4$) and OVA-specific CD8 T cells ($1 \times 10^6$) derived from OT-1 mice. After 72 hours, the culture supernatant was collected and the level of IFN-$\gamma$ therein was measured. The data are expressed as mean $\pm$ standard deviation values.

FIG. 5 shows the expression of PD-L1 on the surface of mouse CT26 colorectal cancer cells. In FIG. 5a, total RNA was obtained from each type of cell, and the PD-L1 transcript was detected by RT-PCR. Mouse liver cancer cells (Hepa-1) were used as a control. In FIG. 5b, PD-L1 protein on the surface of each type of cell was analyzed by flow cytometry.

FIG. 6 shows the *in vivo* and *in vitro* anti-tumor activities of Ad5mTR.sPD1. In FIG. 6(a), CT26 cells were infected with various MOIs of Ad5MOCK, Ad5mTR or Ad5mTR.sPD1 together with 100 $\mu$M GCV. After 3 days, cytotoxicity was measured using the method shown in FIG. 1d. The data were expressed as mean $\pm$ standard deviation for three independent experiments. In FIG. 6(b), both flanks of BALB/c mice were subcutaneously inoculated with CT26 cells ($1 \times 10^6$). The tumor was treated with $5 \times 10^8$ PFU of Ad5MOCK, Ad5EF1$\alpha$.sPD1, Ad5mTR or Ad5mTR.sPD1. The mice were intraperitoneally injected with GCV (75 mg/kg) twice a day. The tumor volume was measured at the indicated time points and recorded. The data are expressed as mean $\pm$ SEM (n=10).

FIG. 7 shows that the inhibition of tumor growth by sPD1-Ig is mediated by CD8 T cells. In FIG. 7(a), a subcutaneous CT26 tumor in BALB/c mice was injected with Ad5MOCK, Ad5mTR or Ad5mTR.sPD1 (left panel). In order to determine the implication of CD8 T cells in the anti-tumor activity of sPD1-Ig, a subcutaneous CT26 tumor in C57/BL6 mice was injected with Ad5MOCK, Ad5mTR or Ad5mTR.sPD1 (right panel). At 2 days before treatment with virus and at 5-day intervals after treatment with virus, 2.43$\alpha$ anti-CD8 antibody (500 $\mu$g) was injected intravenously to deplete CD8 T cells (right panel). The arrow indicates the injection time of the antibody. The tumor volume was measured at the indicated time points and recorded. In FIG. 5(b), the inhibition of tumor growth in the presence or absence of 2.43$\alpha$ anti-CD8 antibody was calculated relative to the value for Ad5MOCK at about 12 days. The data are expressed as mean $\pm$ SEM.

FIG. 8 shows the inhibitory effect of sPD1-Ig on tumor growth in T and B cell-depleted Rag1-/- mice. In FIG. 8(a), an E.G7 tumor injected subcutaneously into C57/BL6 was injected with Ad5MOCK, Ad5mTR or Ad5mTR.sPD1 (left panel). In order to measure the implication of lymphocytes in the anti-tumor activity of sPD1-Ig, an E.G7 tumor injected subcutaneously into Rag1-/- mice was injected with Ad5MOCK, Ad5mTR or Ad5mTR.sPD1 (right panel). The tumor volume was measured at the indicated time points and reported. In FIG. 8(b), a reduction in tumor volume in Rag1-/- mice or wild-type mice was calculated relative to the value for Ad5MOCK at 12 days. In FIG. 9(c), The Rag1-/- mouse E.G7 tumor ($1 \times 10^6$ cells) was injected with $5 \times 10^8$ PFU of Ad5MOCK, Ad5mTR or Ad5mTR.sPD1 twice at 7-day intervals. CD8 T cells ($5 \times 10^4$) from OT-1 mice were intravenously injected at the same time as the first injection of the adenovirus. The tumor volume was measured at the indicated time points and recorded. The data are expressed as mean ±SEM (n=6). In FIG. 8(d), in order to perform the in *vitro* analysis of immune cells, the Rag1-/- mouse E.G7 tumor was co-cultured with OT-1 mouse CD8 T cells and a suitable adenovirus. At 8 days after the first adenovirus injection, PBMCs (peripheral blood mononuclear cells) were collected from the ocular veins of the mice. By flow cytometry, the number of OT-1 CD8 T cells in the total viable cells was determined, and the ratio of TCR V$\alpha$2+, V$\beta$5+, T cells (OT-1 cells) was determined.

FIG. 9 shows the inhibitory effect of treatment with Ad5mTR.sPD1 against a secondary tumor. In FIG. 9(a), a CT26 tumor was transplanted subcutaneously into BALB/c mice, and the mice were injected with $5 \times 10^8$ PFU of Ad5mTR.sPD1 three times at 3-day intervals. 2 Weeks after the first injection, $1 \times 10^6$ tumor cells were introduced into the opposite flank. From 1 day after the first virus injection, GCV (75 mg/kg) was injected intraperitoneally for 12 days. The tumor volume was measured at 3-day intervals. In FIG. 9(b), the E.G7 tumor was transplanted subcutaneously into C57/BL6 mice, and then the same method described above with respect to FIG. 9(a) was performed. In FIG. 9(c), at 7 days after inoculation with the secondary tumor, the tumor volume in mice treated with Ad5mTR.sPD1 and untreated mice was measured. The data are expressed as mean ± standard deviation.

**Best Mode**

In one aspect, the present invention provides a recombinant adenovirus including: a polynucleotide encoding a trans-splicing ribozyme-HSVtk (Human Simplex Virus thymidine kinase) complex, which acts against TERT (Telomerase Reverse Transcriptase) mRNA (SEQ ID NO: 1) that is a cancer-specific gene; and a cancer therapeutic gene.

As used herein, the term "cancer-specific gene" refers to a gene that is expressed specifically in cancer cells or significantly overexpressed in cancer cells. The cancer-specific gene may have characteristics on which the ribozyme according to the present invention can act. This cancer-specific gene may be TERT (telomerase reverse transcriptase) mRNA, AFP (alphafetoprotein) mRNA, CEA (carcinoembryonic antigen) mRNA, PSA (prostate-specific antigen) mRNA, or CKAP2 (Cytoskeleton-associated protein 2) mRNA, but is not limited thereto.

As used herein, the term "TERT (telomerase reverse transcriptase)" refers to one of the most important enzymes, which regulate the immortality and proliferation ability of cancer cells and forms telomeres that function to inhibit cell aging by protecting the chromosomal ends. In normal cells, the length of telomeres decreases little by little whenever the cells divide, and as a result, genetic material is lost and the cells die. However, in cancer cells, this enzyme continuously extends telomeres, and thus the cells do not die. Also, this enzyme is known as an important obstacle in cancer treatment, which contributes directly to the immortality of cancer cells. This telomerase is characterized in that it has a telomerase activity of 80-90% in germ cells, hematopoietic cells and cancer cells, but it has no telomerase activity in normal cells surrounding cancer cells. For the purpose, the TERT can be targeted directly by the cancer therapeutic gene according to the present invention, but is not limited thereto.

As used herein, the term "ribozyme" refers to an enzymatically active RNA molecule having trans-splicing activity and self-splicing activity. For the purpose of the present invention, the ribozyme can serve to inhibit the activity of the cancer-specific gene by a trans-splicing reaction, thereby exhibiting a selective anticancer effect. In addition, it can be expressed in combination with the cancer therapeutic gene to activate the cancer therapeutic gene. Thus, any ribozyme may be used in the present invention, as long as it can inactivate the cancer-specific gene and activate the cancer therapeutic gene. Preferably, it may be either Rib67 ribozyme that is a hTERT-targeting trans-splicing group I ribozyme verified to have trans-splicing ability by recognizing cancer-specific TERT (telomerase reverse transcriptase or a ribozyme that is encoded by a polynucleotide having a nucleotide sequence of SEQ ID NO: 2, but is not limited thereto.

As used herein, the term "HSVtk (herpes simplex virus-thymidine kinase)" refers to thymidine phosphorylase derived from herpes simplex virus. This enzyme is a typical drug-sensitizing gene that allows a non-toxic prodrug into a toxic form so as to kill cells transfected with the gene. For the purpose of the present invention, the HSVtk gene may be used as a cancer-therapeutic gene that is expressed in combination with the ribozyme to exhibit anticancer activity. This HSVtk gene may preferably have a nucleotide sequence set forth in SEQ ID NO: 3 and may be one set forth in Genbank accession Nos. AAP13943, P03176, AAA45811, P04407, Q9QNF7, KIBET3, P17402, P06478, P06479, AAB30917, P08333, BAB84107, AAP13885, AAL73990, AAG40842, BAB11942, NP_044624, NP_044492, CAB06747 or the like, but is not limited thereto.

As used herein, the term "cancer therapeutic gene" refers to a polynucleotide sequence encoding a polypeptide that exhibits a therapeutic effect when being expressed in cancer cells. In the present invention, the cancer therapeutic gene can be expressed along or in combination with the ribozyme to exhibit anticancer activity. Examples of a cancer therapeutic gene that may be used in the present invention include, but are not limited to, a drug-sensitizing gene, a proapoptotic gene, a cytostatic gene, a cytotoxic gene, a tumor suppressor gene, an antigenic gene, a cytokine gene, an anti-angiogenic gene and the like. In the present invention, these cancer therapeutic genes may be used alone or in combination of two or more.

As used herein, the term "drug-sensitizing gene" refers to an enzymatic gene that converts a nontoxic prodrug into a toxic form. It is also referred to as a suicide gene, because cells transfected with the gene die. That is, when a prodrug that is non-toxic in normal cells is systemically administered, it is converted into toxic metabolites only in cancer cells by the drug-sensitizing gene to change drug sensitivity to thereby kill the cancer cells. Typical examples of a drug-sensitive gene that may be used in the present invention include, but are not limited to, a HSV-tk (herpes simplex virus-thymidine kinase) gene, ganciclovir, an *E. coli* cytosine deaminase (CD) gene, 5-fluorocytosine (5-FC), etc.

As used herein, the term "proapoptotic gene" refers to a nucleotide sequence that is expressed to induce programmed cell death. Examples of the proapoptotic gene that is used in the present invention include, but are not limited to, p53, adenovirus E3-11.6K (derived from Ad2 and Ad5) or adenovirus E3-10.5K (derived from Ad), adenovirus E4 gene, p53 pathway gene, and caspase-coding gene.

As used herein, the term "cytostatic gene" refers to a nucleotide sequence that is expressed in cells to stop the cell cycle. Typical examples of a cytostatic gene that may be used in the present invention include, but are not limited to, p21, retinoblastoma gene, E2F-Rb fusion protein gene, cyclin-dependent kinase inhibitor-encoding genes (e.g., p16, p15, p18 and p19), growth arrest specific homeobox (GAX) genes, and the like.

As used herein, the term "cytotoxic gene" refers to a nucleotide sequence that is expressed in cells to exhibit a toxic effect. Examples of the cytotoxic gene that is used in the present invention include, but are not limited to, nucleotide sequences that encode Pseudomoas exotoxin, lysine toxin, diphtheriae toxin and the like.

As used herein, the term "tumor suppressor gene" refers to a nucleotide sequence that can be expressed in target cells to inhibit tumor phenotypes or induce cell death. Examples of a tumor suppressor gene that may be used in the present invention include, but are not limited to, tumor necrosis factor-$\alpha$ (TNF-$\alpha$), p53 gene, APC gene, DPC-4/Smad4 gene, BRCA-1 gene, BRCA-2 gene, WT-1 gene, retinoblastoma gene, MMAC-1 gene, adenomatous polyposis coil protein, deleted colorectal carcinoma (DCC) gene, MMSC-2 gene, NF-1 gene, ENT tumor suppressor gene located in chromosome 3p21.3, MTS1 gene, CDK4 gene, NF-1 gene, NF-2 gene, VHL gene, sPD-1 (soluble programmed death-1), etc.

As used herein, the term "sPD-1 (soluble programmed death-1)" refers to the extracellular domain of PD-1 (programmed death-1) known as a co-inhibitory molecule on T cells, which is a member of the family of immunoglobulin molecules. In general, the interaction between PD-1 and PD-L1 induces a decrease in the level of tumor invasive lymphocytes and in the T cell receptor-mediated proliferation and causes the immune evasion of tumor cells. However, recent studies reported that sPD-1 that is a soluble form of PD-1 can effectively induce anticancer immune responses by inhibiting immune evasion caused by the interaction between PD-1 and PD-L1. Thus, for the purpose of the present invention, the sPD-1 gene can be conjugated to the cancer-specific gene by the trans-splicing activity of the ribozyme according to the present invention so as to be used as a therapeutic gene against cancer cells. Preferably, the sPD-1 gene may have a nucleotide sequence set forth in SEQ ID NO: 4, but is not limited thereto.

As used herein, the term "antigenic gene" refers to a nucleotide sequence which is expressed in target cells to produce a cell surface antigenic protein that can be recognized in the immune system. Examples of an antigenic gene that may be used in the present invention include, but are not limited to, carcinoembryonic antigen (CEA), p53 and the like.

As used herein, the term "cytokine gene" refers to a nucleotide sequence which is expressed in cells to produce cytokine. Examples of a cytokine gene that may be used in the present invention include, but are not limited to, GM-CSF, interleukins (IL-1, IL-2, IL-4, IL-12, IL-10, IL-19 and IL-20), interferon $\alpha$, $\beta$ and $\gamma$ (interferon $\alpha$-2b), and fusions such as interferon $\alpha$-2$\alpha$-1.

As used herein, the term "anti-angiogenic gene" refers to a nucleotide sequence which is expressed in cells to release anti-angiogenic factors out of the cells. Examples of an anti-angiogenic gene that may be used in the present invention include, but are not limited to, angiostatin, inhibitors of vascular endothelial growth factor (VEGF), endostatin, and the like.

As used herein, the term "adenovirus" has the same meaning as an adenovirus vector and refers to a member of the family *Adenoviridae.* The *Adenoviridae* includes all animal adenoviruses of the genus *Mastadenovirus.* In particular, human adenoviruses include the A-F subgenera and the individual serotypes thereof. The A-F subgenera includes, but is not limited to, human adenovirus types 1, 2, 3, 4, 4a, 5, 6, 7, 8, 9, 10, 11 (Ad11A and Ad11P), 12, 13, 14, 15, 16, 17, 18, 19, 19a, 20, 21, 22, 23, 24, 25, 26, 27, 28, 29, 30, 31, 32, 33, 34, 34a, 35, 35p, 36, 37, 38, 39, 40, 41, 42, 43, 44, 45, 46, 47, 48 and 91.

In another aspect, the present invention provides a pharmaceutical composition for preventing or treating cancer, which contains the above-described recombinant adenovirus as an active ingredient.

The recombinant adenovirus according to the present invention includes a polynucleotide encoding a trans-splicing ribozyme HSVtk complex, which acts against the cancer-specific gene TERT, and a cancer therapeutic gene. Thus, when the recombinant adenovirus is administered to cancer cells in which TERT is expressed, HSVtk can be dissociated from the trans-splicing ribozyme-HSVtk complex due to TERT, and the dissociated HSVtk can exhibit cytotoxicity and induce the attach of the cancer therapeutic gene against the cancer cells. Thus, the recombinant adenovirus can function as a cancer therapeutic agent. However, when the recombinant adenovirus is administered to normal cells in which TERT is not expressed, HSVtk will not exhibit cytotoxicity, because HSVtk is not dissociated from the trans-splicing ribozyme-HSVtk complex. Accordingly, the recombinant adenovirus of the present invention shows high selectivity for cancer cells, and thus can be used for cancer therapy in a safer manner.

As used herein, the term "cancer" refers to cells or tissue in which cells have abnormally proliferated due to abnormalities in the function of regulating the division, differentiation and death thereof and invaded the surrounding tissue and organ to form a mass and destroy or modify existing structures. Examples of this cancer include, but are not limited to, pancreatic cancer, breast cancer, brain tumors, head and neck carcinoma, melanoma, myeloma, leukemia, lymphoma, liver cancer, stomach cancer, colon cancer, bone cancer, uterine cancer, ovarian cancer, rectal cancer, esophageal cancer, small intestine cancer, anal cancer, fallopian tube carcinoma, endometrial carcinoma, cervical cancer, vaginal carcinoma, vulva cancer, Hodgkin's disease, bladder cancer, renal cancer, ureteral cancer, renal cell carcinoma, renal pelvis carcinoma, and central nervous system tumors.

As used herein, the term "preventing" refers to all actions that inhibit cancer or delay the development of cancer by administering the recombinant adenovirus or composition of the present invention.

As used herein, the term "treating" refers to all actions that alleviate or beneficially change cancer by administering the recombinant adenovirus or composition of the present invention.

In addition, the pharmaceutical composition of the present invention may further comprise a pharmaceutically acceptable carrier, excipient or diluent.

Examples of a pharmaceutically acceptable carrier, excipient or diluent that may be used in the pharmaceutical composition of the present invention include lactose, dextrose, sucrose, sorbitol, mannitol, xylitol, erythritol, maltitol, starch, acacia gum, alginate, gelatin, calcium phosphate, calcium silicate, calcium carbonate, cellulose, methyl cellulose, polyvinyl pyrrolidone, water, methyl hydroxybenzoate, propyl hydroxylbenzoate, talc, magnesium stearate, mineral oil, etc.

The pharmaceutical composition of the present invention may be formulated according to conventional methods in oral dosage forms, including powders, granules, tablets, capsules, suspensions, emulsions, syrup and aerosol, preparations for external application, suppositories, and sterile injectable solutions. The composition of the present invention may be formulated with commonly used diluents or excipients, such as fillers, extenders, binders, wetting agents, disintegrants, surfactants, etc. Solid formulations for oral administration include tablets, pills, powders, granules, capsules and the like, and such solid formulations comprise, in addition to the recombinant adenovirus, at least one excipient, for example, starch, calcium carbonate, sucrose, lactose or gelatin. In addition to simple excipients, lubricants such as magnesium stearate or talc may also be used. Liquid formulations for oral administration include suspensions, solutions, emulsions, and syrup, and may contain various excipients, for example, wetting agents, flavoring agents, aromatics and preservatives, in addition to water and liquid paraffin, which are frequently used simple diluents. Formulations for parenteral administration include sterilized aqueous solutions, non-aqueous solutions, suspensions, emulsions, freeze-dried preparations, and suppositories. As non-aqueous solvents or suspending agents, propylene glycol, polyethylene glycol, plant oils such as olive oil, injectable esters such as ethyl oleate, and the like can be used. As the base of the suppositories, witepsol, Macrogol, Tween 61, cacao butter, laurin fat, glycerogelatin and the like can be used.

In another aspect, the present invention provides a method for treating cancer, which comprises administering a pharmaceutically effective amount of the above-described recombinant adenovirus or pharmaceutical composition to a subject in need of treatment.

As used herein, the term "pharmaceutically effective amount" refers to an amount sufficient to treat diseases, at a reasonable benefit/risk ratio applicable to any medical treatment. The effective dosage level of the composition may be determined depending on the subject's type, the disease severity, the subject's age and sex, the type of infected virus, the activity of the drug, sensitivity to the drug, the time of administration, the route of administration, excretion rate, the duration of treatment, drugs used in combination with the composition, and other factors known in the medical field. The pharmaceutical composition of the present invention may be administered individually or in combination with other therapeutic agents, and may be administered sequentially or simultaneously with conventional therapeutic agents. The composition of the present invention can be administered in a single or multiple dosage form. It is important to administer the composition in the minimum amount that can exhibit the maximum effect without causing side effects, in view of all the above-described factors, and this amount can be easily determined by a person skilled in the art. Specifically, the pharmaceutical composition of the present invention is preferably administered orally or intravenously.

As used herein, the term "administering" means introducing a predetermined substance into an animal by any suitable method. The pharmaceutical composition of the present invention may be administered by any general route, as long as it can reach a target tissue. In addition, the pharmaceutical composition of the present invention may be administered using any system capable of delivering the active ingredient to target cells.

The preferred dosage of the composition according to the present invention may vary depending on the patient's conditions and weight, the severity of disease, the type of formulation, the route of administration and the duration of treatment, but may be selected appropriately by a person skilled in the art. However, for desired effects, the composition of the present invention may be administered in a daily dosage of 1 to 10 mg/kg, and preferably 1 to 5 mg/kg. The daily dosage may be taken in a single dose, or may be divided into several doses.

The pharmaceutical composition of the present invention may be used alone or in combination with auxiliary therapeutic methods such as surgical therapy. Chemotherapeutic agents that may be used together with the composition of the present invention include, but are not limited to, cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin, methotrexate, and the like. In addition, radiotherapies that may be used together with the composition of the present invention include, but are not limited to, X-ray irradiation and γ-ray irradiation.

In an example of the present invention, the present inventors a trans-splicing ribozyme such that a ribozyme that recognizes and cleaves human TERT (hTERT) is linked with HSVtk mRNA and cleaved at the 3' end of hTERT mRNA (FIG. 1). Also, in order to confirm the effect of the trans-splicing ribozyme in mice, the present inventors designed a similar system using a trans-splicing ribozyme that targets mouse TERT mRNA (mTERT-TR), and the designed trans-splicing ribozyme was inserted into an E1/E3-deleted adenovirus genome to construct an adenovirus (Ad5mTR) including an mTERT-TR-controlled HSVtk (mTERT-TR-HSVtk) (FIG. 2a). The constructed adenovirus was infected into CT26 colorectal cancer cells derived from BALB/c mice, and as a result, it was found that when the cells were infected with 2.5 MOI of the adenovirus, most of the cells could be killed (FIG. 2b), and that when Ad5mTR was injected into a subcutaneous CT26 tumor in BALB/c mice, the growth of the tumor was significantly inhibited, similar to cytotoxicity *in vitro* (FIG. 2c). In addition, the present inventors tested the ability of Ad5mTR to induce cell death in an E.G7 tumor injected subcutaneously into B6 mice, and as a result, it was shown that a tumor antigen was released after infection with the virus (FIG. 3a) and that DCs derived from Ad5mTR-treated mice could provide a sufficient level of OVA antigen to stimulate OVA-specific T cells (FIG. 3b).

Meanwhile, the present inventors constructed a dual-module adenovirus (Ad5mTR.sPD1) comprising HSVtk, which is controlled by mTER-TR in the E1 region of the adenovirus genome, and sPD1-Ig present in the E3 region (FIG. 4a), and tested the activity of the dual-module adenovirus. As a result, it was found that the secretion of IFN-γ by antigen-specific T cells in response to the attack of tumor cells was increased by the supernatant containing sPD1-Ig (FIG. 4d) and that the expression of PD-L1 on the surface of tumor cells was confirmed by flow cytometry (FIG. 5). In addition, it was shown that CT26 cells infected with Ad5mTR.sPD1 showed *in vitro* cytotoxicity similar to that of CT26 cells infected with Ad5mTR (FIG. 6a) and that Ad5mTR.sPD1 significantly promoted tumor regression as compared to Ad5mTR and resulted in almost complete tumor regression (FIG. 6b). As expected in the present invention, HSVtk promoted the responsiveness of antigen-specific CD8 T cells by DCs (FIG. 3b), and sPD1-Ig promoted the responsiveness of anti-tumor CD8 T cells in an extracellular environment (FIG. 4d).

The improved inhibitory effect of this dual-module Ad5mTR.sPD1 on tumor growth was almost lost in CD8 T cell-depleted mice (FIG. 7a), and the effect of depletion of CD8 on anti-tumor activity was higher for Ad5mTR.sPD1 than for Ad5mTR (1.9-fold versus 4.76-fold) (FIG. 7b). In addition, in the CT26 model, the effect of Ad5mTR.sPD1 was stronger than that of Ad5mTR (1.61-fold versus 6.58-fold) (FIG. 8b), and when antigen-specific T cell response was formed by OT-I T cells injected intravenously into Rag1-/- mice containing E.G7, the anti-tumor effect of administration of the adenovirus was restored, and this effect was more evident upon administration of Ad5mTR.sPD1 compared to administration of Ad5mTR (FIG. 8c). Furthermore, the number of OT-I T cells in blood significantly increased upon administration of Ad5mTR.sPD1 compared to administration of Ad5mTR (FIG. 8d).

This effect of the adenovirus was analyzed *in vivo,* and as a result, it was shown that, in mice administered with Ad5mTR.sPD1, the proliferation of the primary tumor was minimized and the secondary tumor did not grow (FIGS. 9a and 9c), and the same results were observed in E.G7 tumor-bearing B6 mice (FIGS. 9b and 9c).

Although this effect of the recombinant adenovirus according to the present invention was observed in mice, the recombinant adenovirus according to the present invention can also be used for treatment of human cancer, because TERT against which the adenovirus is effective is expressed not only in mouse tumors, but also in human tumors.

## Mode for Invention

Hereinafter, the present invention will be described in detail with reference to preferred examples. It is to be understood, however, that these examples are for illustrative purposes only and are not intended to limit the scope of the present invention.

<u>Example 1: Materials and method</u>

<u>Example 1-1: Cells and mice</u>

All cells were cultured in RPMI medium containing 10% FBS (fetal bovine serum) and 1% penicillin/streptomycin. 6-week old female BALB/c and C57/BL6 mice were purchased from SLC (Japan). OT-1 mice (B6 background) and Rag1-/- mice (B6 background) were obtained from Jackson Laboratory. All animal studies were performed in accordance with the Guidelines for the Care and Use of Laboratory Animals of the National Cancer Center (Korea).

**Example 1-2: Construction of adenovirus**

In order to generate Ad5mTR containing mouse TERT-TR-HSVtk gene under the control of CMV promoter, AdenoZAPTM and AdenoQuickTM systems were used. pAVQ-CMV-mTERT AS100 Rib(+67) TK was treated with SpeI/SacII to obtain a DNA fragment, and the fragment was inserted into the SpeI/EcoRV cleavage site of pZAP1.1, thereby constructing pZAP1.1.CMV.mTR.HSVtk. The pZAP1.1.CMV.mTR.HSVtk was digested with PacI/DraIII, ligated with RightZAP1.2, and then introduced into HEK293 cells. In order to construct sPD1-Ig, the extracellular domain of PD-1 was amplified by PCR using the following primers:

Forward primer: 5'-CCG CTC GAG CTC ACC ATG TGG GTC CGG CAG GTA CCC TGG-3' (SEQ ID NO: 5)
Reverse primer: 5'-AGA TCT TCC TCC TCC TCC TTG AAA CCG GCC TTC TGG TTT GGG-3' (SEQ ID NO: 6)

The amplification product was inserted into the XhoI/BglII site of a pFUSE-mIgG2A.Fc1 vector (Invitrogen, San Diego, CA) to construct pFUSE-mIgG2A.Fc1.EF1.sPD-1. EF1.sPD1-Ig derived from pFUSE-mIgG2A.Fc1.EF1.sPD1-Ig was inserted into the EcoRI/SwaI site of a pE3.1 vector (OD260) to construct pE3.1.EF1.sPD1-Ig. The CMV.mTR.HSVtk fragment of pZAP1.1.CMV.mTR.HSVtk was inserted into the BamHI/SpeI site of a pE1.2 vector (OD260) to construct pE1.2.CMV.mTR.HSVtk. In order to construct Ad5mTR.sPD1, pE1.2.CMV.mTR.HSVtk and pE3.1.EF1.sPD1-Ig were digested with the restriction enzyme DraIII/PflMI, ligated with AdenoQuick13.1, and then introduced into HEK293 cells. In order to construct Ad5EF1.sPD1, in the same manner as the construction of Ad5mTR.sPD1, pE3.1.EF1.sPD1-Ig and a pE1.2 empty vector were digested with the restriction enzyme DraIII/PflMI, ligated with AdenoQuick13.1, and then introduced into HEK293 cells.

Example 1-3: GCV absorption and cytotoxicity analysis in extracellular environment

The enzymatic activity of HSVtk was determined by measuring the accumulation of phosphorylated GCV in cells. Cell proliferation assay (Dojindo Laboratories, Rockville, MD) was performed by evaluating the cytotoxicity of the adenovirus using a standard method. In summary, cells ($3 \times 10^3$) were inoculated into a 96-well plate and cultured overnight at 37 °C. The cultured cells were infected with various MOIs (multiplicity of infections) of the adenovirus. After 1 day, GCV was added to the cells to a final concentration of 200 $\mu$M, and then the proliferation of the cells was measured for 3 days. All the experiments were repeated three times.

Example 1-4: Antibody and reagent

In order to examine the expression of sPD1-Ig protein from Ad5CMV.mTR.sPD1, HEK293 cells ($4 \times 10^5$) were infected with 2 MOI of the adenovirus. At 24 hours after the infection, the culture supernatant and the cell residue (80 $\mu$g) were analyzed by immunoblotting using anti-Pdcd-1 antibody (Santa Cruz Biotechnology, Santa Cruz, CA). In order to deplete CD8 T cells, 500 $\mu$g of 2.43$\alpha$ anti-CD8 antibody was injected intraperitoneally 2 days before infection with the adenovirus, after which the antibody was injected intraperitoneally at 5-day intervals for 15 days.

**Example 1-5: Analysis of CD8 T cell/DC coculture for analyzing the production of IFN-$\gamma$**

Draining lymph node-derived DCs (DLN-DCs) were separated by 17.5% Nycodenz gradient and purified on an MACS column using anti-CD11c microbeads (Miltenyi Biotec, Auburn, CA). Pure CD8 T cells derived from OT-1 mice were separated using anti-CD8 microbeads. DLN-DCs ($5 \times 10^4$) were co-cultured with pure CD8 T cells ($1 \times 10^5$) in a 96-well plate for 72 hours. After 72 hours, the culture supernatant was collected, and the content of IFN-$\gamma$ therein was measured by ELISA (eBioscience, San Diego, CA).

**Example 1-6: Analysis of OT-1 T cell activation in extracellular environment**

Mouse colorectal cancer MC38/OVA cells (B6 background) that stably express ovalbumin (OVA) were cultured in a 6-well plate. sPD1-Ig was collected from the culture supernatant of HEK293 infected with 10 MOI of the adenovirus for 24 hours, after which it was added to MC38/OVA cells ($1 \times 10^4$) to which OT-1 CD8 T lymphocytes ($1 \times 10^6$) were added, followed by culture for 72 hours. Pure CD8+ T lymphocytes derived from OT-1 mice were separated using an MACS column as described above. The amount of IFN-$\gamma$ produced from the CD8 T lymphocytes was measured using a mouse IFN-$\gamma$ CBA assay kit (BD bioscience, San Jose, CA).

Example 1-7: In vivo animal study and in vitro analysis

1 × $10^6$ CT26 cells were inoculated subcutaneously into 6-week old female BALB/c mice. When a tumor was sensed (after 7 days), 5 × $10^8$ PFU of the adenovirus was injected into the tumor, and GCV (75 mg/kg) was injected intravenously twice a day for 14 days. The adenovirus was administered twice at 7-day intervals. The tumor volume was determined using the following equation:

$$\text{Length} \times \text{width}^2 \times 0.5236$$

Similar procedures for subcutaneous tumor formation and virus injection were performed in a CD8 T cell depletion experiment. E.G7 cells (C57/BL6 background; 1 × $10^6$) were injected into Rag1-/- mice or C57/BL6 mice and treated with the adenovirus and GCV according to the above-described method. For *in vitro* analysis, The Rag1-/- mouse E.G7 tumor was treated with Ad5mTR.sPD1 according to the method of intravenously administering CD8 T cells separated from OT-1 mice. At 8 days after the infection, PBMCs (peripheral blood mononuclear cells) were collected from the blood and analyzed by flow cytometry.

Example 2: Results

Example 2-1: Construction of adenovirus comprising mouse TERT-TR-HSVtk

The present inventors developed a novel HSVtk expression strategy using the known tumor marker TERT and a tumor-specific adenovirus. In this strategy, a ribozyme that recognizes and cleaves human TERT (hTERT) was delivered to tumor cells using a recombinant adenovirus. Additionally, the ribozyme was designed such that it is linked with HSVtk mRNA and cleaved at the 3' end of hTERT mRNA. It induced the novel translation of HSVtk mRNA in tumor cells. This kind of ribozyme is defined as trans-splicing ribozyme (FIG. 1). Theoretically, the expression of HSVtk that is regulated by the trans-splicing ribozyme is limited to cells, including tumor cells, which express hTERT mRNA at high levels. Thus, this system was proposed as an effective method of delivering HSVtk to tumor cells without influencing the surrounding normal tissue cells in which hTERT is expressed at high levels. Indeed, this strategy showed a high effect against human colorectal cancer cells transplanted into a xenograft mouse model. One problem of this system in immunological analysis was that the experiment using human tumor cells was performed in immunodeficient mice in which xenograft rejection was inhibited. Thus, this system is not suitable for evaluating the effect of HSVtk on anti-tumor immunization. To overcome this limitation, the present inventors designed a similar system using a trans-splicing ribozyme that targets mouse TERT mRNA (mTERT-TR), and the immunological effect of TERT-TR-controlled HSVtk was evaluated using a mouse tumor model. The TERT recognition sequence is located near the HSVtk-encoding sequence, and the expression of HSVtk is dependent on the presence of mTERT transcripts (FIG. 1). This expression cassette that is controlled by CMV promoter was inserted into an E1/E3-deleted adenovirus genome to construct an adenovirus (Ad5mTR) comprising an mTERT-TR-controlled HSVtk (mTERT-TR-HSVtk) (FIG. 2a).

As a control, an adenovirus (Ad5MOCK) that does not comprise the expression cassette was constructed. In order to examine whether the expression of HSVtk by Ad5mTR shows cytotoxicity in mouse tumor cells, the present inventors used CT26 colorectal cancer cells derived from BALB/c mice, and such cells express mTERT mRNA at high levels. CT26 cells were exposed to various MOIs of Ad5mTR. 2.5 MOI of Ad5mTR was enough to kill most of the cells, whereas Ad5MOCK showed no cytotoxicity even at 50 MOI (FIG. 2b). When Ad5mTR was injected into a subcutaneous CT26 tumor in BALB/c mice, the growth of the tumor was significantly inhibited, similar to cytotoxicity *in vitro* (FIG. 2c).

Thus, it can be seen that Ad5mTR shows an anti-tumor effect similar to that of an adenovirus comprising human TERT-TR-HSVtk and is suitable for use in anti-tumor immunogenicity in mice.

Example 2-2: Improved DC-mediated antigen presentation by HSVtk

It is known that the expression of HSVtk in tumor cells by DNA introduction or adenovirus infection promotes the antigenic response of cytotoxic CD8 T cells. Because the expression of HSVtk in the presence of GCV *in vivo* can induce cell death in a significant portion of the tumor volume, a tumor antigen derived from killed cells can be detected by APCs such as DCs, and these cells migrate to lymph nodes that response to tumor antigen-specific T cells. Although this model was suggested in the literature, the present inventors decided to test this possibility using the defined antigen-specific T cells. For this test, the present inventors used the mouse tumor cell line E.G7 (a derivative of EL4 cells that stably expresses ovalbumin) as a model antigen tumor) and purified T cells from OVA-specific T cell receptor transgenic mice used as a model tumor antigen (OVA)-specific CD8 T cell colony. All the CD8 T cells purified from the OT-1 mice were OVA-specific cytotoxic T cells. First, the present inventors tested the ability of Ad5mTR to induce cell death in an E.G7 tumor injected subcutaneously into B6 mice. Histological evaluation was performed after separation of the tumor and administration of Ad5mTR, and as a result, a significant level of cell death was observed, suggesting that a tumor antigen was released after infection with the virus (FIG. 3a). Then, the present inventors purified DCs from tumor-draining lymph nodes and evaluated whether OVA was loaded for IFN-γ production, by analysis of a co-culture of DC/CD8 T cells. OT-I T cells, cultured and purified with DCs derived from tumor-bearing mice treated with Ad5mTR, produced a larger amount of IFN-γ compared to OT-I T cells cultured with DCs derived from tumor-bearing mice treated with a control virus. This result suggests that DCs derived from Ad5mTR-treated mice can provide a sufficient level of OVA antigen to stimulate OVA-specific T cells (FIG. 3b). Thus, it can be seen that the expression of HSVtk in the tumor and the resulting GCV-induced cell death resulted from the release of tumor antigens and that these antigens were efficiently captured by DCs that can stimulate tumor antigen-specific cytotoxic T cells.

From these results, it could be seen that the tumor-specific expression of HSVtk can effectively stimulate anti-tumor T cell activity by DCs and can directly induce cytotoxicity in tumor cells.

Example 2-3: Construction of adenovirus including both mTERT-TR-HSVtk and sPD1-Ig

Ad5mTR stimulated the anti-tumor CD8 T cell responsiveness with an increasing possibility of interest. A combination of these strategies having another strategy for inactivating tumor-induced immune resistance further enhanced the responsiveness of anti-tumor T cells. PD-L1 is a known immune suppressor that is expressed on the surface of tumor cells. The present inventors constructed sPD1 (a soluble form of PD-L1 receptor that neutralizes PD-L1) and removed PD-L1-mediated T cell suppression. In order to increase the stability of sPD1 *in vivo,* sPD1 was fused with the Fc region of IgG2a to construct sPD1-Ig. The present inventors constructed a dual-module adenovirus (Ad5mTR.sPD1) including HSVtk, which is controlled by mTERT-TR in the E1 region of the adenovirus genome, and sPD1-Ig present in the E3 region (FIG. 4a). HEK293 cells were infected with Ad5mTR.sPD1, sPD1-Ig was expressed and secreted in an extracellular environment and analyzed by immunoblotting (FIG. 4b). The expression level of HSVtk in the Ad5mTR.sPD1-infected cells was determined by measuring the enzymatic activity and was compared with that in Ad5mTR-infected cells (FIG. 4c). In addition, the present inventors tested whether sPD1-Ig secreted in an *in vitro* environment can enhance the responsiveness of anti-tumor T cells by neutralizing PD-L1 on the tumor cell surface. A co-culture of OVA-expressing tumor cells and OVA-specific OT-I T cells was added to the culture supernatant of the Ad5EF1α.sPD1-infected 293HEK, and the responsiveness of the T cell cells was measured based on the secretion of IFN-γ. As expected, the secretion of IFN-γ by the antigen-specific T cell in response to the attack of the tumor cells was increased by the sPD1-Ig-containing supernatant (FIG. 4d). The expression of PD-L1 on the tumor cell surface was confirmed by flow cytometry (FIG. 5). These results suggest that the dual-module can enhance the responsiveness of anti-tumor T cells in the tumor microenvironment *in vivo.*

Example 2-4: Effective inhibition of tumor growth by Ad5mTR.sPD1

To confirm whether sPD1-Ig can increase the anti-tumor activity of HSVtk *in vivo,* a CT26 colorectal cancer model was used (FIG. 2c). This model was selected because CT26 cells express a high level of PD-L1 on the surface thereof (FIG. 5). It was expected that CT26 cells infected with Ad5mTR.sPD1 would show cytotoxicity similar to that of CT26 cells infected with Ad5mTR and that sPD1-Ig would not be the direct cause of cytotoxicity caused by HSVtk (FIG. 6a). In comparison with this, Ad5mTR.sPD1 significantly promoted tumor regression compared to Ad5mTR, and almost tumor regression was observed in the case of Ad5mTR.sPD1 (FIG. 6b). A control adenovirus (Ad5EF1α.sPD1) comprising sPD1-Ig alone did not show any anti-tumor effect in this model, suggesting that the expression of sPD1-Ig in tumor tissue is not sufficient for inducing tumor regression and that an antigen response by HSVtk *in vivo* is necessary for the effect of sPD1-Ig.

Example 2-5: Tumor growth inhibitory effect of sPD1-Ig is mediated by CD8 T cells

The HSVtk promoted the response of antigen-specific CD8 T cells by DCs (FIG. 3b), and sPD1-Ig promoted the responsiveness of anti-tumor CD8 T cells in an extracellular environment (FIG. 4d). Such results suggest that the enhanced responsiveness of CD8 T cells *in vivo* is attributable to the enhanced anti-tumor effect of Ad5mTR.sPD1. To confirm this possibility, anti-CD8 antibody was administered into CT26 tumor-bearing mice to deplete CD8 T cells. The enhanced tumor growth inhibitory effect of the dual-module Ad5mTR.sPD1 was almost lost in the CD8 T cell-depleted mice (FIG. 7a). Interestingly, the anti-tumor activity of Ad5mTR was also reduced, suggesting that the effect of Ad5mTR in the CT26 mouse tumor model is dependent on anti-tumor immune responses mediated by CD8 T cells. However, the effect of depletion of CD8 on anti-tumor activity was higher for Ad5mTR.sPD1 than for Ad5mTR (1.9-fold versus 4.76-fold) (FIG. 7b). To more directly evaluate the function of tumor-specific CD8 T cells, E.G7 tumor models and OVA-specific OT-I T cells were used. The subcutaneous injection of an E.G7 tumor into B6 mice administered with Ad5mTR.sPD1 showed tumor regression, similar to that observed in the CT26 model. In comparison with this, when the same experiment was performed on lymphocyte-deleted Rag1-/- mice, the anti-tumor effects of the two types of viruses were significantly reduced (FIG. 8a). Similar to the effect of depletion of CD8 T cells, the degree of tumor growth reduction in the CT26 model was higher for Ad5mTR.sPD1 than for Ad5mTR (1.61-fold versus 6.58-fold) (FIG. 8b). When antigen-specific T cell response was formed by OT-I T cells injected intravenously into Rag1-/- mice bearing E.G7, the anti-tumor effect of administration of the adenovirus was restored, and this effect was more evident upon administration of Ad5mTR.sPD1 compared to administration of Ad5mTR (FIG. 8c). Furthermore, the number of OT-I T cells in blood significantly increased upon administration of Ad5mTR.sPD1 compared to administration of Ad5mTR (FIG. 8d). Such results suggest that Ad5mTR.sPD1 promotes tumor regression by directly enhancing the function of tumor-specific CD8 T cells.

Example 2-6: Anticancer effect of treatment with Ad5mTR.sPD1

In the blood of the mice administered with Ad5mTR.sPD1 having an increased possibility of inhibiting the growth of the secondary tumor in the distal portion of the primary tumor region injected with the virus, the number of cancer-specific T cells increased. Ad5mTR.sPD1 was administered to a CT26 tumor in BALB/c mice three times at 3-day intervals. At 14 days after the first administration, the generation of a secondary CT26 tumor in the opposite flank of the same mice was challenged. In this case, in the mice administered with Ad5mTR.sPD1, the proliferation of a primary tumor was minimized, whereas in the mice administered with Ad5MOCK, a primary tumor having a large size (> 600 $mm^3$) was generated. Thus, a new group of normal BALB/c mice was used as a control group for generation of a secondary tumor. In the control group, the secondary tumor smoothly grew in the control group, but did not grow in the mice administered with Ad5mTR.sPD1 (FIGS. 9a and 9c). The same result was observed in E.G7 tumor-bearing B6 mice (FIGS. 9b and 9c). Thus, it can be seen that injection of the dual-module adenovirus provides an anticancer effect by enhancing anticancer immunity.

## Claims

1. A recombinant adenovirus comprising: a polynucleotide encoding a trans-splicing ribozyme-HSVtk (Human Simplex Virus thymidine kinase) complex, which acts against TERT (telomerase reverse transcriptase) mRNA (SEQ ID NO: 1) that is a cancer-specific gene; and a cancer-therapeutic gene.

2. The recombinant adenovirus of claim 1, wherein the polynucleotide encoding the complex comprises a nucleotide sequence of SEQ ID NO: 2, which encodes Rib67 ribozyme, and a polynucleotide of SEQ ID NO: 3, which encodes HSVtk.

3. The recombinant adenovirus of claim 1, wherein the cancer therapeutic gene is selected from the group consisting of a drug-sensitizing gene, a proapoptotic gene, a cytostatic gene, a cytotoxic gene, a tumor suppressor gene, an antigenic gene, a cytokine gene, and an anti-angiogenic gene.

4. The recombinant adenovirus of claim 3, wherein the cancer-therapeutic gene comprises a nucleotide sequence of SEQ ID NO: 4, which encodes sPD-1 (soluble Programmed Death-1).

5. A pharmaceutical composition for preventing or treating cancer, the composition comprising the recombinant adenovirus of any one of claims 1 to 4 as an active ingredient.

6. The composition of claim 5, further comprising a pharmaceutically acceptable carrier, excipient or diluent.

7. A method for treating cancer, the method comprising administering a pharmaceutically acceptable amount of the recombinant adenovirus of any one of claims 1 to 4 to a subject in need of treatment.

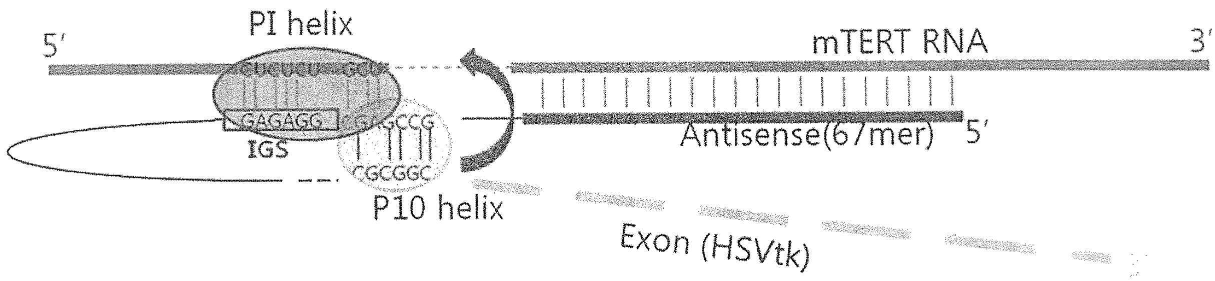
PI helix
5′
mTERT RNA
3′
CUCUCU
GCU
GAGAGG
CGAGCCG
IGS
Antisense(67mer)
5′
CGCGGC
P10 helix
Exon (HSVtk)

Fig. 1

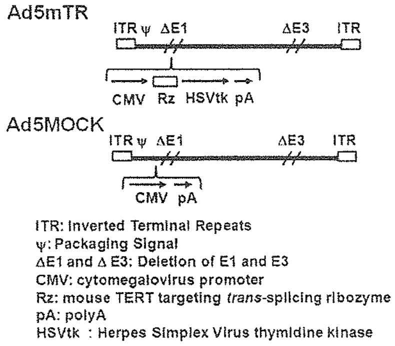


Fig. 2a

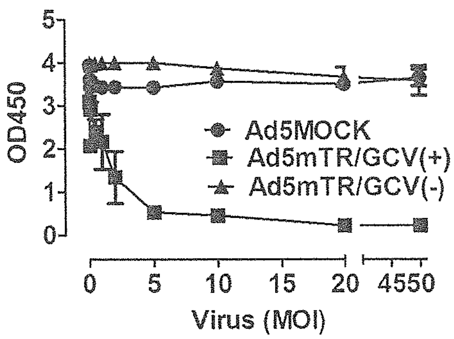


Fig. 2 b

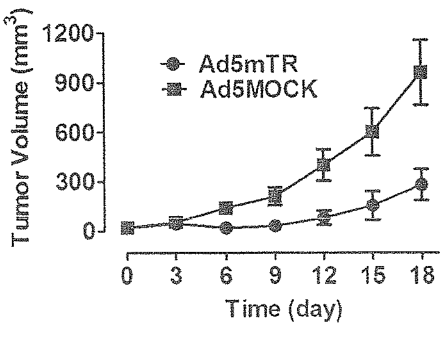


Fig. 2 c

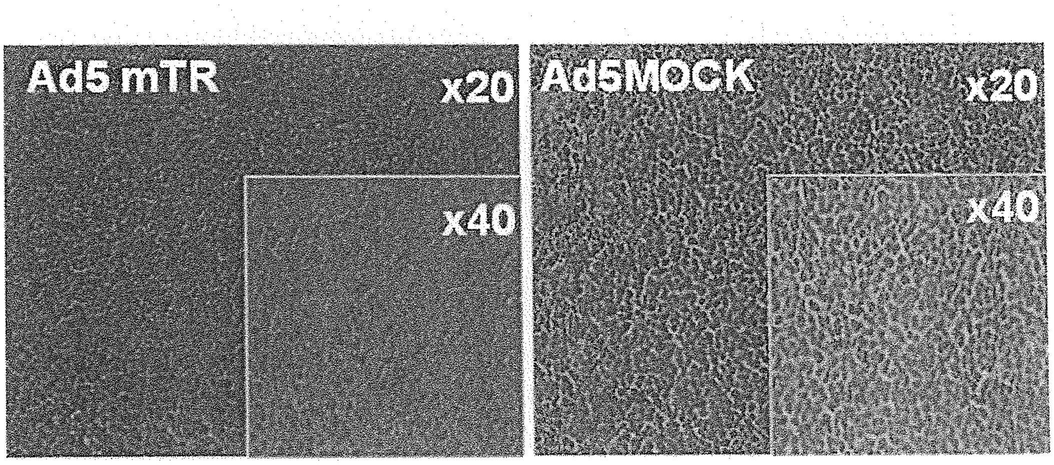
Ad5 mTR
x20
x40
Ad5MOCK
x20
x40

Fig. 3a

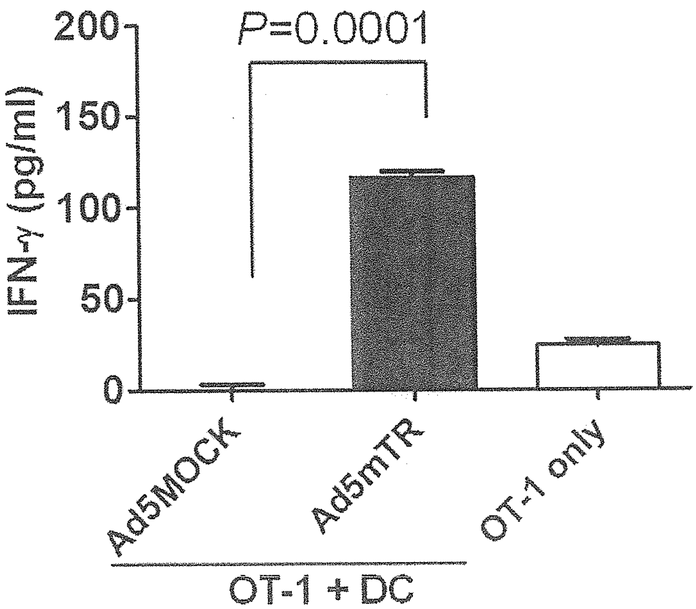
P=0.0001
IFN-γ (pg/ml)
200
150
100
50
0
Ad5MOCK
Ad5mTR
OT-1 only
OT-1 + DC

Fig. 3b

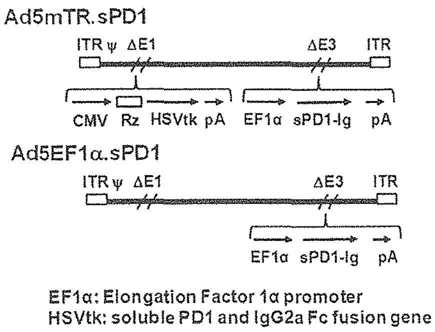


Fig. 4a

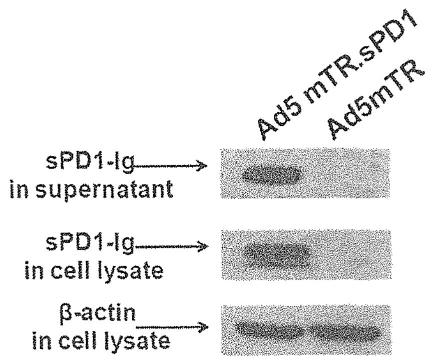


Fig. 4b

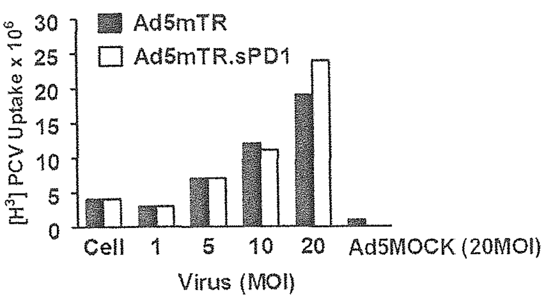


Fig. 4c

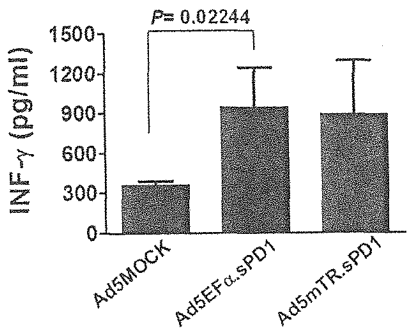


Fig. 4d

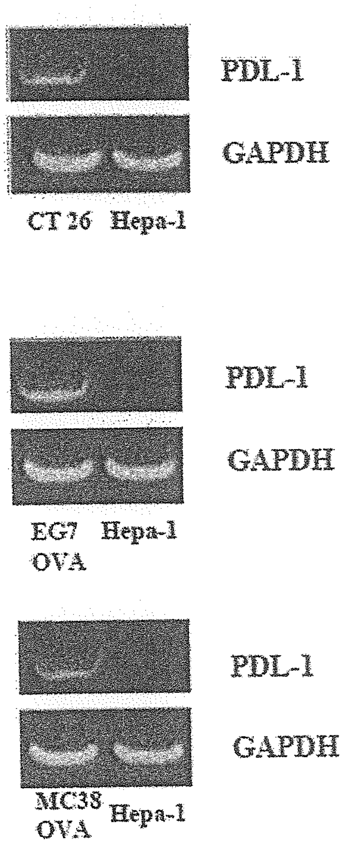
PDL-1
GAPDH
CT 26 Hepa-1
PDL-1
GAPDH
EG7 OVA Hepa-1
PDL-1
GAPDH
MC38 OVA Hepa-1

Fig. 5a

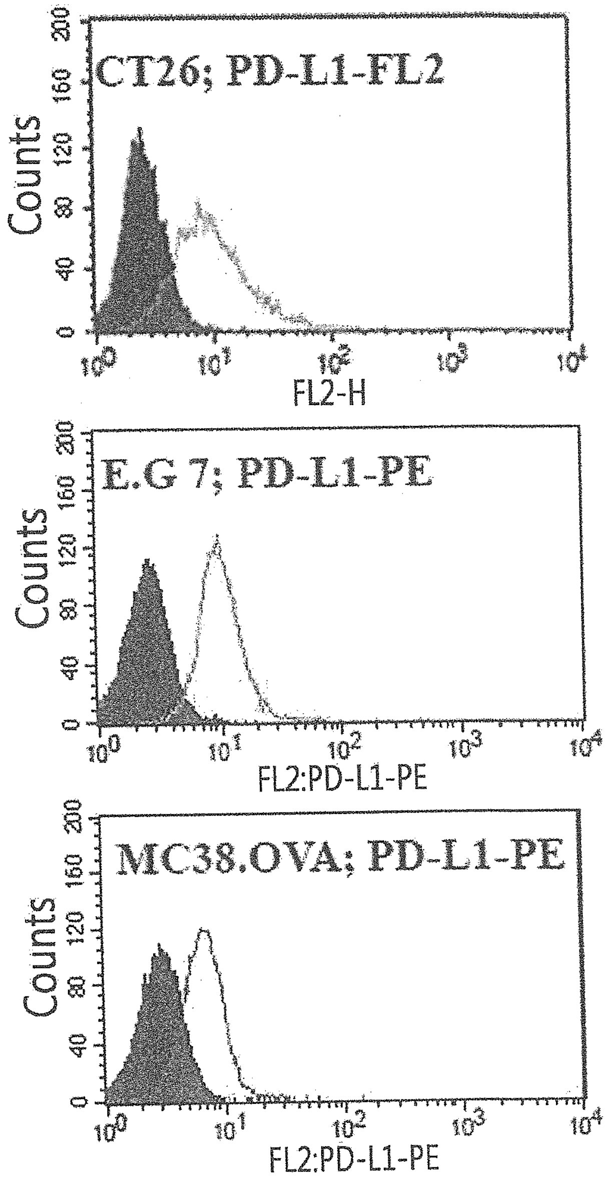
CT26; PD-L1-FL2
Counts
FL2-H
E.G 7; PD-L1-PE
Counts
FL2:PD-L1-PE
MC38.OVA; PD-L1-PE
Counts
FL2:PD-L1-PE

Fig. 5b

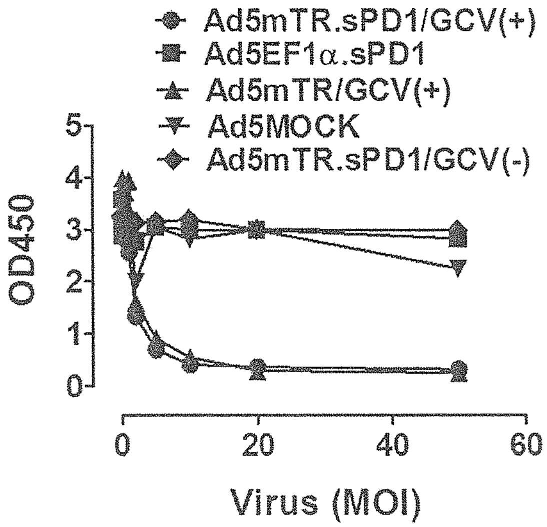
Ad5mTR.sPD1/GCV(+)
Ad5EF1α.sPD1
Ad5mTR/GCV(+)
Ad5MOCK
Ad5mTR.sPD1/GCV(-)
OD450
0
1
2
3
4
5
0
20
40
60
Virus (MOI)

Fig. 6a

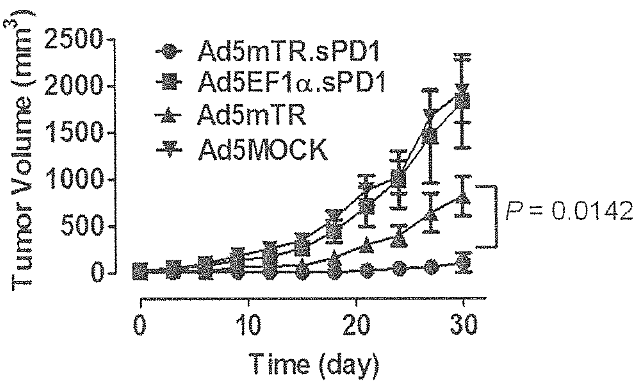
Tumor Volume (mm3)
2500
2000
1500
1000
500
0
Ad5mTR.sPD1
Ad5EF1α.sPD1
Ad5mTR
Ad5MOCK
P = 0.0142
0
10
20
30
Time (day)

Fig. 6b

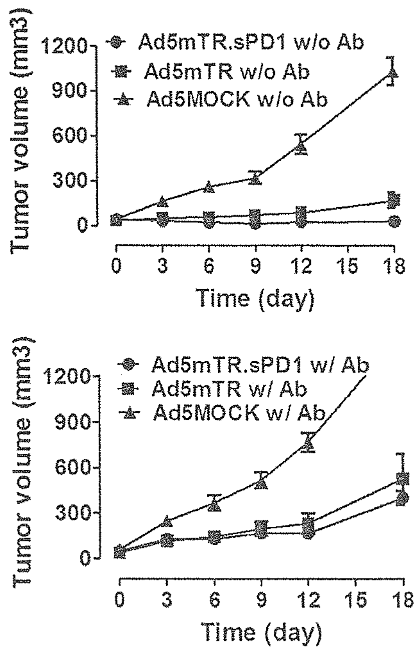
Tumor volume (mm3)
1200
900
600
300
0
Ad5mTR.sPD1 w/o Ab
Ad5mTR w/o Ab
Ad5MOCK w/o Ab
0 3 6 9 12 15 18
Time (day)
Tumor volume (mm3)
Ad5mTR.sPD1 w/ Ab
Ad5mTR w/ Ab
Ad5MOCK w/ Ab
Time (day)

Fig. 7a

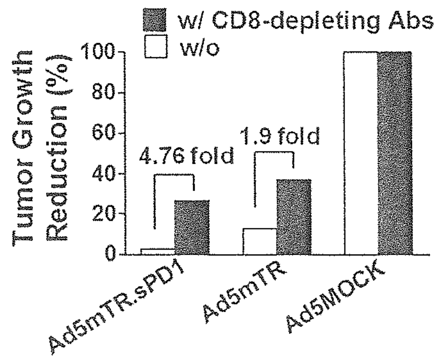
w/ CD8-depleting Abs
w/o
Tumor Growth Reduction (%)
100
80
60
40
20
0
4.76 fold
1.9 fold
Ad5mTR.sPD1
Ad5mTR
Ad5MOCK

Fig. 7b

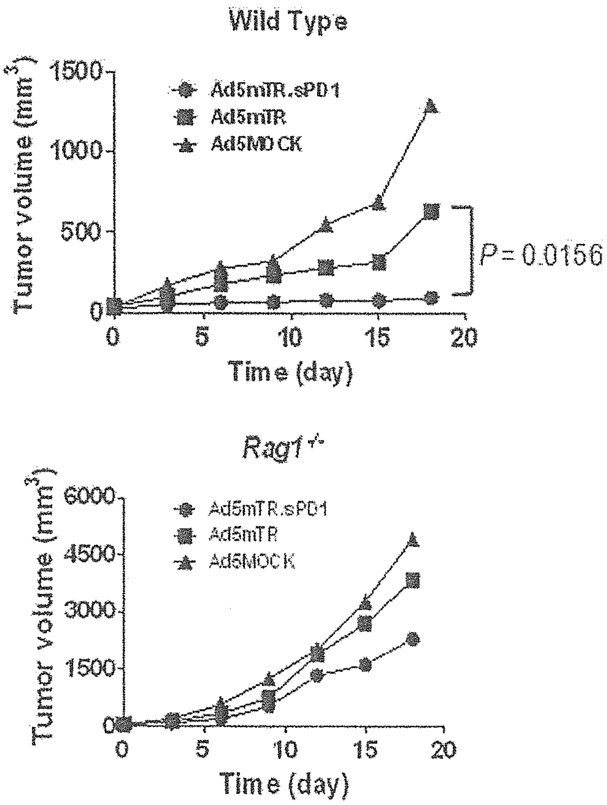


Fig. 8a

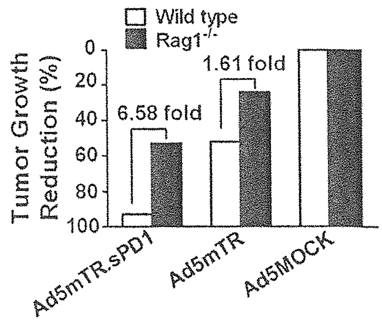


Fig. 8b

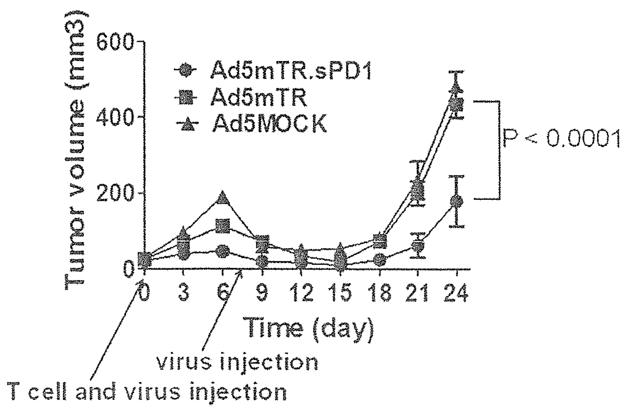


Fig. 8c

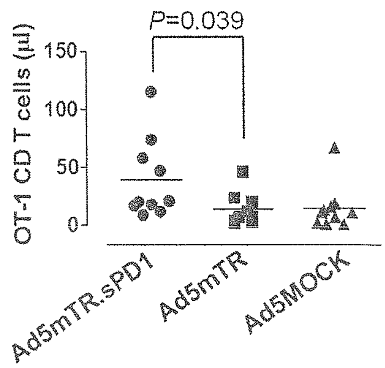


Fig. 8d

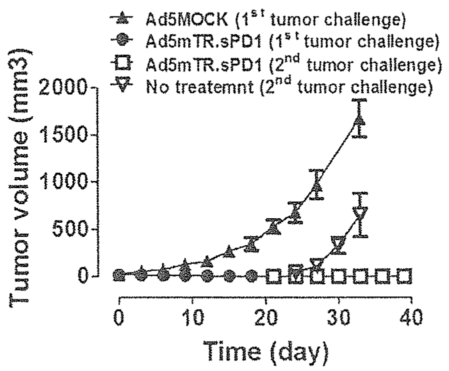


Fig. 9a

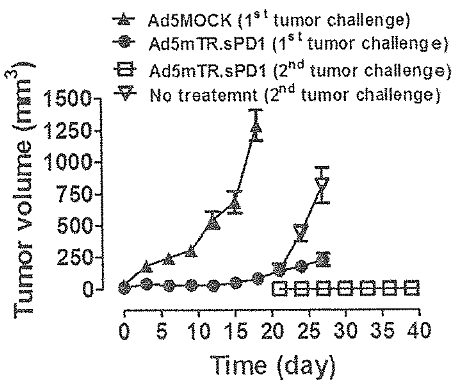


Fig. 9b

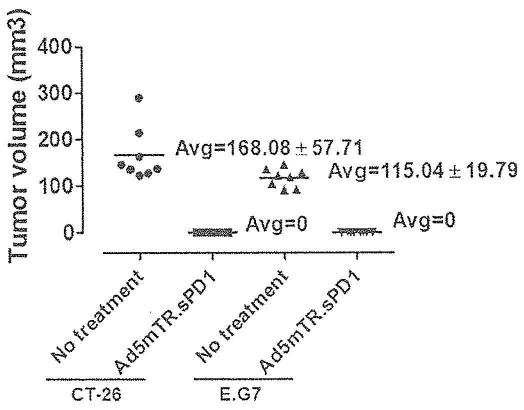


Fig. 9c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9007936 A
- US 5837510 A
- US 5861290 A
- WO 9804290 A
- WO 9737542 A
- US 5631236 A


### Non-patent literature cited in the description


- **FUKUZAWA et al.** *Cancer Res,* 2004, vol. 64, 363-369